(19) Europäisches Patentamt
European Patent Office
Office européen des brevets

(11) **EP 3 423 136 B1**

(12) **EUROPÄISCHE PATENTSCHRIFT**

(45) Veröffentlichungstag und Bekanntmachung des Hinweises auf die Patenterteilung:
**04.08.2021 Patentblatt 2021/31**

(21) Anmeldenummer: **17702107.8**

(22) Anmeldetag: **31.01.2017**

(51) Int Cl.:
**A61M 16/00** *(2006.01)*

(86) Internationale Anmeldenummer:
**PCT/EP2017/052001**

(87) Internationale Veröffentlichungsnummer:
**WO 2017/148639 (08.09.2017 Gazette 2017/36)**

(54) **VORRICHTUNG ZUR BEATMUNG EINES PATIENTEN**

DEVICE FOR VENTILATING A PATIENT

DISPOSITIF DE VENTILATION D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.03.2016 DE 102016103678**
**24.05.2016 DE 102016109528**

(43) Veröffentlichungstag der Anmeldung:
**09.01.2019 Patentblatt 2019/02**

(73) Patentinhaber: **Ventinova Technologies B.V.**
**5652 BJ Eindhoven (NL)**

(72) Erfinder: **ENK, Dietmar**
**48653 Coesfeld (DE)**

(74) Vertreter: **Rössler, Matthias**
**karo IP Patentanwälte**
**Kahlhöfer Rößler Kreuels PartG mbB**
**Postfach 32 01 02**
**40416 Düsseldorf (DE)**

(56) Entgegenhaltungen:
| EP-A1- 0 776 672 | WO-A1-00/41757 |
|---|---|
| WO-A1-2016/206979 | WO-A2-01/68162 |
| DE-B3-102005 039 220 | JP-A- 2001 095 919 |
| US-A- 5 303 698 | US-A1- 2010 108 066 |
| US-A1- 2011 023 881 | |

**Beschreibung**

Der Gegenstand der vorliegenden Erfindung bezieht sich auf eine Beatmungsvorrichtung zur Beatmung eines Patienten. Die Vorrichtung umfasst zumindest eine Fluidzuführeinheit und zusätzlich eine Fluidabführeinheit, die zum Zuführen eines Fluids (Atemgas) in zumindest einen Atemweg, also in einen Lungenteil oder in die Lunge, eines Patienten oder zum Abführen des Fluids aus diesem Atemweg geeignet ist.

Bei der Beatmung eines Patienten wird normalerweise eine Maske oder ein Tubus eingesetzt, über die bzw. den dem nach außen abgedichteten Atemweg mit niedrigem Druck ein Gas oder Gasgemisch, insbesondere Sauerstoff und Luft, zugeführt wird. Alternativ kann man ein solches Gas oder Gasgemisch aber auch mit hohem Druck und hohem Fluss stoßweise durch einen dünnen, ungeblockten Katheter in den nach außen offenen Atemweg injizieren (sogenannte Jet-Ventilation). Dieses Verfahren wird heute insbesondere bei diagnostischen und therapeutischen Eingriffen im Bereich des oberen Atemweges eingesetzt (endotracheale oder transtracheale Jet-Ventilation). Dabei kann dieses Verfahren in Notfallsituationen auch außerhalb von Krankenhäusern oder in stationären Situationen in Krankenhäusern angewendet werden.

Bei der transtrachealen Jet-Ventilation kann ein Patient durch einen durch die Haut direkt in die Luftröhre eingebrachten Katheter bzw. eine so platzierte Kanüle mit Sauerstoff bzw. einem Fluid versorgt werden. Diese Verfahren (trans-/endotracheal) sind Bestandteil der heute gültigen Algorithmen zum Management eines schwierigen Atemweges und insbesondere der Situation, in der ein Patient konventionell nicht zu beatmen bzw. zu intubieren ist (sogenannte "cannot ventilate, cannot intubate"-Situation).

Weiterhin sind aus der WO 2008/113752 A1 und WO 2015/004229 A1 jeweils Gasstromumkehr-Vorrichtungen bekannt, mit denen eine Beatmung (Inspiration und Exspiration) auch ausschließlich über einen Katheter erfolgen kann.

Künstliche oder mechanische Beatmung erfolgt entweder kontrolliert oder in Form von unterstützter Spontanatmung. Im ersten Fall hat das Beatmungsgerät (Respirator) die vollständige Kontrolle über das Atemmuster, während im zweiten Fall der zumindest teilweise spontan atmende Patient wesentlichen Einfluss auf das Atemmuster hat. Allen Formen der Beatmung ist aber gemeinsam, dass das Beatmungsgerät nahezu ausschließlich Einfluss auf die Einatemphase (Inspiration) nimmt. Die Exspiration kann - aus der Perspektive des Respirators - passiv erfolgen, d. h. die in den elastischen Gewebeelementen von Lunge und Thorax gespeicherte Energie treibt die Exspiration an.

Es sind verschiedene Beatmungsverfahren bekannt. Üblicherweise wird eine volumenkontrollierte Beatmung durchgeführt, wobei sämtliche Beatmungsparameter vorgegeben werden. Ziel- und Steuerungsparameter ist das Tidalvolumen (Atemzugvolumen $V_T$). Die resultierenden Atemwegsdrücke sind abhängig von den eingestellten Volumina sowie den pulmonalen Gegebenheiten des Patienten. Einstellparameter sind also Volumenstrom, Beatmungsfrequenz und PEEP (positive endexpiratory pressure). Der positive endexspiratorische Druck bezeichnet einen bei der Beatmung künstlich in der Lunge erzeugten positiven Druck, der nach Abschluss der Ausatmung (Exspiration) anliegt.

Bei der druckkontrollierten Beatmung wird ein anfangs hoher Volumenstrom erst bei Erreichen eines eingestellten hohen inspiratorischen Druckniveaus reduziert. Zielparameter und Kontrollvariable ist also der Druck. Eine Einstellung des Volumenstroms ist hierbei also nicht möglich.

Im Gegensatz zur Spontanatmung eines Patienten wird bei der künstlichen Beatmung das Fluid gegen die Elastizität des Atemwegs zugeführt. Durch die Erhöhung des Drucks im Brustraum verringert PEEP den Rückfluss des venösen Blutes zum Herzen, wodurch das Herzzeitvolumen sinken kann. Umgekehrt entsteht ein Rückstau in oberer und unterer Hohlvene mit entsprechenden Druckerhöhungen in vorgeschalteten Organen. Abhängig von der Höhe des PEEP kann es dadurch zu Schädigungen und Funktionseinschränkungen von Gehirn, Leber, Nieren und anderer Organe kommen.

Die EP 0776 672 A1 ist auf ein Verfahren zur Kontrolle einer Beatmungsvorrichtung gerichtet. Dabei soll die Zufuhr von Beatmungsluft hin zum Patienten gesteuert werden.

Die US 2010/0108066 A1 ist auf ein Verfahren zur Steuerung einer Atmung eines Patienten während einer Schlafphase gerichtet. Über eine für das Verfahren eingesetzte Vorrichtung wird nur ein Volumenstrom hin zum Patienten geregelt.

Hiervon ausgehend liegt der vorliegenden Erfindung die Zielsetzung zugrunde, eine Beatmungsvorrichtung vorzuschlagen, die es ermöglicht, ein verbessertes Beatmungsverfahren durchzuführen. Insbesondere soll die Beatmung möglichst individuell abgestimmt erfolgen, d. h. die Eigenschaften des zu beatmenden Patienten sollen vollumfänglich berücksichtigt werden. Weiterhin soll die Beatmung möglichst schonend erfolgen, eine Schädigung der Atemwege und anderer Organe soll in jedem Fall verhindert werden.

Diese Zielsetzung wird durch Beatmungsvorrichtungen mit den Merkmalen der Patentansprüche 1 und 9 erreicht. Vorteilhafte Weiterbildungen und Ausgestaltungen der Beatmungsvorrichtungen sind Gegenstand der jeweils abhängigen Ansprüche. Es ist darauf hinzuweisen, dass die in den abhängig formulierten Ansprüchen einzeln aufgeführten Merkmale in technologisch sinnvoller Weise miteinander kombiniert werden können und weitere Ausgestaltungen der Erfindung definieren. Darüber hinaus werden die in den Ansprüchen angegebenen Merkmale in der Beschreibung näher präzisiert und erläutert, wobei weitere bevorzugte Ausgestaltungen der Erfindung dargestellt werden.

Die Erfindung betrifft eine (erste) Beatmungsvorrichtung zur Beatmung eines Patienten, zumindest umfassend eine Fluidzuführeinheit und zusätzlich eine Fluidabführeinheit, die zum Zuführen eines Fluids in zumindest einen Atemweg, also in einen Lungenteil oder in die Lunge, eines Patienten und zum Abführen des Fluids aus diesem Atemweg geeignet ist. Weiter umfasst die Beatmungsvorrichtung eine Steuereinrichtung, die, während einer Beatmung des zumindest einen Atemwegs des Patienten, also dem Zuführen eines Fluids in den zumindest einen Atemweg und/oder dem Abführen des Fluids aus dem zumindest einen Atemweg, durch Betreiben der Beatmungsvorrichtung zur Ermittlung oder zusätzlich zum Abschätzen eines Verlaufs zumindest eines Teilbereichs einer Compliance-Kurve des mindestens einen Atemwegs geeignet ist. Die Ermittlung oder die zusätzliche Abschätzung eines Verlaufs zumindest eines Teilbereichs einer Compliance-Kurve erfolgt durch das Zuführen und/oder Abführen des Fluids aus dem zumindest einen Atemweg und durch Ermittlung zumindest eines Wertes der Compliance.

Für die Compliance gilt: C = V / delta p [Milliliter/Millibar].

Die Compliance gibt an, wie viel Fluid, also ein Volumen V [Milliliter], in den mindestens einen Atemweg eingeführt oder aus dem Atemweg entfernt wird, so dass sich ein Druck in dem Atemweg um eine Druckdifferenz delta p [Millibar] verändert. Die Steuereinrichtung bestimmt, unter Berücksichtigung des ermittelten oder zusätzlich abgeschätzten Verlaufs des zumindest einen Teilbereichs der Compliance-Kurve, eine Lage eines Druckintervalls mit den Drücken P1 und P2 und stellt diese Drücke an der Beatmungsvorrichtung ein, so dass zumindest ein Beatmungsvorgang, also eine Inspiration und eine Exspiration, zwischen diesen Drücken P1 und P2 erfolgt und ein Betrag der Compliance dieses Beatmungsvorgangs möglichst groß ist.

Der Betrag gibt den Wert der Compliance unabhängig von dessen Vorzeichen an. Der Grundgedanke der Erfindung ist es, die Beatmung des Patienten mit einem möglichst geringen Energieeintrag durchzuführen. Ein geringer Energieeintrag in die Atemwege des Patienten bedeutet auch eine geringstmögliche Schädigung der Atemwege und weiterer Organe des Patienten. Eine solche Minimierung des Energieeintrags wird durch die Ermittlung des geringstmöglichen Druckes erreicht, bei dem ein benötigtes Atemzugvolumen $V_T$ (Tidalvolumen) dem Patienten zugeführt werden kann. Diese Drücke P1 und P2 des Druckintervalls werden anhand der jeweils vorliegenden Compliance des beatmeten Patienten ermittelt.

Insbesondere wird also während mindestens eines Beatmungsvorganges (Inspiration und/oder Exspiration) der Verlauf der Compliance-Kurve ermittelt oder zusätzlich abgeschätzt (z. B. aufgrund von Erfahrungswerten). Dabei wird insbesondere gerade der Teilbereich der Compliance-Kurve ermittelt, bei dem ein bestimmtes Volumen V (ggf. $V_T$) in einem möglichst kleinen Druckintervall zugeführt werden kann.

Insbesondere wird zur Ermittlung des Verlaufs der Compliance-Kurve ein Volumen an Fluid, bevorzugt ein kleines Volumen von höchstens 100 ml, besonders bevorzugt von höchstens 50 ml, über die Fluidzuführeinheit dem mindestens einen Atemweg zugeführt. Während und/oder bevorzugt nach Zuführung dieses Volumens wird die Druckänderung delta p in dem mindestens einen Atemweg gemessen und ein Wert für die Compliance ermittelt. Entweder wird nun bereits unter Berücksichtigung von Erfahrungswerten oder ggf. unter Berücksichtigung von bereits vorher ermittelten Werten für die Compliance dieses Patienten zumindest der Verlauf des Teilbereichs der Compliance-Kurve abgeschätzt. Oder es werden weitere (kleine) Volumen zugeführt die jeweilige Druckänderung delta p ermittelt. Aus diesen Werten für die Compliance kann (mit steigender Genauigkeit) der Verlauf zumindest des Teilbereichs der Compliance-Kurve bestimmt und/oder abgeschätzt werden. Weiterhin kann aufgrund von fallenden oder steigenden Beträgen der Werte der Compliance der Verlauf der Compliance-Kurve und die bevorzugte Lage eines, für die folgende Beatmung des Patienten vorgesehenen Druckintervalls mit den Drücken P1 und P2 bestimmt bzw. abgeschätzt werden.

Insbesondere kann zumindest eine der folgenden Größen: PEEP, Atemfrequenz, Volumenstrom voreingestellt werden, so dass das benötigte Atemzugvolumen $V_T$, unter der Randbedingung eines möglichst geringen Energieeintrags, zugeführt werden kann. Bevorzugt kann auch jede dieser Größen nach Ermittlung und Bewertung des Beatmungsvorganges weiter angepasst werden, so ein vorbestimmtes Atemzugvolumen $V_T$ unter den dann eingestellten Parametern zugeführt wird.

Eine Fluidzuführeinheit und ggf. auch eine Fluidabführeinheit umfasst zumindest eine Druckgasquelle bzw. eine Einrichtung, mit der ein Fluid (z. B. ein Gas bzw. ein Gasgemisch, geeignet zur Sicherstellung der Beatmung eines Patienten) in den bzw. aus dem mindestens einen Atemweg des Patienten eingeführt bzw. ausgeführt werden kann. Bevorzugt ist ausschließlich eine Druckgasquelle vorhanden, bzw. erfolgt auch die Ausatmung über eine eingangs erwähnte Gasstromumkehr-Vorrichtung, wobei das Fluid über ein Lumen in den Atemweg eingeführt und über das gleiche Lumen wieder abgeführt wird.

Die Steuereinrichtung ist zur Ermittlung oder zusätzlich zum Abschätzen eines Verlaufs zumindest eines Teilbereichs einer Compliance-Kurve geeignet. Die Ermittlung der Compliance-Kurve erfolgt während einer Beatmung durch das Zuführen und/oder Abführen des Fluids aus dem zumindest einen Atemweg und durch Ermittlung zumindest eines Wertes der Compliance. Der Verlauf der Compliance-Kurve eines Patienten ist insbesondere unter Berücksichtigung des mindestens einen Wertes der Compliance abschätzbar.

Insbesondere greift die Steuereinrichtung dabei auf die Messwerte mindestens eines Drucksensors zurück und überwacht die über die Fluidzuführeinheit zugeführten bzw. über die Fluidabführeinheit abgeführten Volumenströme.

Insbesondere wird der in dem Atemweg jeweils vorliegende Druck überwacht und/oder gemessen bzw. rechnerisch abgeschätzt bzw. ermittelt. Bevorzugt ist also ein Drucksensor in dem Atemweg angeordnet, so dass insbesondere auch eine kontinuierliche Druckmessung in dem Atemweg auch während der Beatmung möglich ist.

Eine derartige Anordnung eines Drucksensors ist insbesondere bei der Ermittlung des Verlaufs der Compliance-Kurve vorteilhaft, da hier bei der kontinuierlichen, oder in Teilschritten erfolgenden Zufuhr eines Volumens oder von Teilvolumina des Fluids der sich (jeweils) ändernde Druck delta p bestimmt werden kann.

Gemäß einer bevorzugten Weiterbildung ermittelt die Steuereinrichtung zumindest während einer Inspiration oder einer Exspiration eines Beatmungsvorgangs eine Mehrzahl von Werten für die Compliance und bestimmt daraus für zumindest einen folgenden Beatmungsvorgang die Lage des Druckintervalls mit den Drücken P1 und P2, für das ein Betrag der Compliance möglichst groß ist. Insbesondere ermittelt die Steuereinrichtung kontinuierlich oder in vorbestimmbaren Zeitabständen die Werte für die Compliance. Bevorzugt werden mindestens 5, besonders bevorzugt mindestens 10 Werte für die Compliance je Beatmungsvorgang bestimmt.

Insbesondere ist die Anzahl der je Beatmungsvorgang zu bestimmenden Werte für die Compliance einstellbar. Bevorzugt wird für eine Inspiration eine andere Anzahl von Werten bestimmt als für eine Exspiration.

Insbesondere werden durch die Steuereinrichtung Werte für die Compliance über eine Mehrzahl von Beatmungsvorgängen hinweg kontinuierlich (also nicht nur während eines Beatmungsvorganges sondern während jedes Beatmungsvorgangs) ermittelt, so dass wahlweise für jeden folgenden Beatmungsvorgang oder für mehrere aufeinanderfolgende Beatmungsvorgänge die Lage des Druckintervalls mit den Drücken P1 und P2 neu bestimmbar ist.

Insbesondere bestimmt die Steuereinheit, in Abhängigkeit von der ermittelten Lage des Druckintervalls und des Druckintervalls selbst sowie der dabei bestimmten Compliance, zumindest für den folgenden Beatmungsvorgang zumindest einen der folgenden Parameter:

- ein Atemzugvolumen (Tidalvolumen) $V_T$ [Milliliter],
- einen Druck P1 und einen Druck P2 [Millibar],
- eine Beatmungsfrequenz F [1/Sekunde],

Gemäß einer bevorzugten Ausgestaltung der Beatmungsvorrichtung ist mindestens ein Drucksensor innerhalb der Atemwege des Patienten anordenbar und der Druck in dem Atemweg ist über eine Messung innerhalb des zumindest einen Atemwegs ermittelbar.

Bevorzugt ist durch die Beatmungsvorrichtung zumindest der Druckanstieg, also delta p / delta t [Millibar/Sekunde] während einer Inspiration kontrollierbar und begrenzbar.

Gemäß einer bevorzugten Ausgestaltung der Beatmungsvorrichtung ist durch die Beatmungsvorrichtung zumindest der Druckabfall, also delta p / delta t [Millibar/Sekunde] während einer Exspiration kontrollierbar und begrenzbar.

Gemäß einer bevorzugten Ausgestaltung der Beatmungsvorrichtung sind der Druckanstieg und der Druckabfall kontrollierbar und begrenzbar.

Bevorzugt ist der Betrag des Druckanstiegs oder Druckabfalls auf höchstens 40 mbar/s [Millibar/Sekunde], insbesondere höchstens 30 mbar/s, bevorzugt höchstens 20 mbar/s begrenzbar.

Es wird weiter ein (erstes) Verfahren zum Betreiben einer Beatmungsvorrichtung, insbesondere der erfindungsgemäßen Beatmungsvorrichtung, vorgeschlagen. Die Beatmungsvorrichtung ist zur Beatmung eines Patienten vorgesehen. Das Verfahren umfasst zumindest die folgenden Schritte:

a) Zuführen eines Fluids in zumindest einen Atemweg, also einen Lungenteil oder die Lunge, des Patienten und/oder Abführen des Fluids aus diesem Atemweg durch Betreiben der Beatmungsvorrichtung;

b) Ermitteln oder zusätzlich Abschätzen eines Verlaufs zumindest eines Teilbereichs einer Compliance-Kurve des mindestens einen Atemwegs durch das Zuführen und/oder Abführen des Fluids in Schritt a) und Ermittlung zumindest eines Wertes der Compliance, wobei für die Compliance gilt:

$$C = V / \text{delta } p \text{ [Milliliter/Millibar]};$$

wobei die Compliance angibt, wie viel Fluid, also ein Volumen V [Milliliter], in den mindestens einen Atemweg eingeführt oder aus dem Atemweg entfernt wird, so dass sich ein Druck in dem Atemweg um eine Druckdifferenz delta p [Millibar] verändert;

c) Bestimmen einer Lage eines Druckintervalls mit den Drücken P1 und P2 entlang des Verlaufs des in Schritt b) ermittelten oder zusätzlich abgeschätzten zumindest einen Teilbereichs der Compliance-Kurve, wobei für ein in diesem Druckintervall durchgeführten Beatmungsvorgang, also eine Inspiration und/oder eine Exspiration, ein Betrag der Compliance möglichst groß ist;

d) Zu- und/oder Abfuhr des Fluids innerhalb des in Schritt c) ermittelten Druckintervalls in zumindest einem auf Schritt c) folgenden Beatmungsvorgang.

Die Ausführungen zu der Beatmungsvorrichtung gelten gleichermaßen für das hier vorgeschlagene Verfahren und umgekehrt.

Es wird also ein Verfahren zur Beatmung eines Patienten mit einem möglichst geringen Energieeintrag vorgeschlagen. Die Minimierung des Energieeintrags wird durch die Ermittlung des geringstmöglichen Druckes erreicht, bei dem ein benötigtes Atemzugvolumen $V_T$ (Tidalvolumen) dem Patienten zugeführt werden kann. Diese Drücke P1 und P2 des Druckintervalls werden anhand der jeweils vorliegenden Compliance des beatmeten Patienten ermittelt.

Insbesondere wird in Schritt b) zumindest während einer Inspiration oder einer Exspiration eines Beatmungsvorgangs eine Mehrzahl von Werten für die Compliance ermittelt, so dass in Schritt c) für zumindest einen folgenden Beatmungsvorgang die Lage des Druckintervalls mit den Drücken P1 und P2 bestimmbar ist, für das ein Betrag der Compliance möglichst groß ist. Insbesondere werden durch die Steuereinrichtung die Werte für die Compliance kontinuierlich oder in vorbestimmbaren Zeitabständen ermittelt. Bevorzugt werden mindestens 5, besonders bevorzugt mindestens 10 Werte für die Compliance je Beatmungsvorgang bestimmt.

Bevorzugt werden die Schritte b), c) und d) kontinuierlich durchgeführt, so dass wahlweise für jeden folgenden Beatmungsvorgang oder für mehrere aufeinanderfolgende Beatmungsvorgänge die Lage des Druckintervalls mit den Drücken P1 und P2 neu bestimmt wird.

Gemäß einer bevorzugten Ausgestaltung wird in Abhängigkeit von der in Schritt c) ermittelten Lage des Druckintervalls und des Druckintervalls selbst sowie der dabei bestimmten Compliance zumindest für den folgenden Beatmungsvorgang zumindest einer der folgenden Parameter bestimmt:

- ein Atemzugvolumen $V_T$ (Tidalvolumen) [Milliliter],
- ein Druck P1 und ein Druck P2 [Millibar],
- eine Beatmungsfrequenz F [1/Sekunde],

Gemäß einer bevorzugten Ausgestaltung wird der Druck über eine Messung innerhalb der Atemwege ermittelt.

Gemäß einer vorteilhaften Ausgestaltung wird zumindest der Druckanstieg, also delta p / delta t [Millibar/Sekunde] während einer Inspiration kontrolliert und begrenzt.

Gemäß einer anderen vorteilhaften Ausgestaltung wird zumindest der Druckabfall, also delta p / delta t [Millibar/Sekunde] während einer Exspiration kontrolliert und begrenzt.

Bevorzugt werden der Druckanstieg und der Druckabfall kontrolliert und begrenzt.

Insbesondere wird der Betrag des Druckanstiegs oder Druckabfalls auf höchstens 40 mbar/s [Millibar/Sekunde], insbesondere höchstens 30 mbar/s, bevorzugt höchstens 20 mbar/s begrenzt.

Insbesondere wird für die Beatmung des Patienten ein Katheter eingesetzt, der einen, zumindest für ein während der Inspiration zugeführtes Fluid, durchströmbaren Querschnitt von höchstens 30 mm$^2$ [Quadratmillimeter] aufweist.

Insbesondere kann über einen solch geringen Querschnitt (bei auschließlicher Inspiration und Exspiration über dieses Lumen) eine Begrenzung des Druckanstiegs während der Inspiration aber auch während der Exspiration erreicht werden.

Insbesondere kann in einer Fluidabführeinheit ein Widerstand (z. B. ein Strömungswiderstand oder ähnliches) vorgesehen sein, der den Druckabfall während der Exspiration begrenzt und kontrolliert.

Für Beatmungsvorrichtung und Verfahren gilt gleichermaßen, dass insbesondere zunächst ein, für den zumindest einen Atemweg des zu beatmenden Patienten vorliegender, Teilbereich einer Compliance-Kurve ermittelt und ggf. zusätzlich abgeschätzt wird. Dafür wird der Druckanstieg während der Zugabe eines bestimmten Volumens V (z. B. 50 oder 100 ml [Milliliter]; ggf. auch $V_T$) gemessen.

Weiter wird insbesondere danach ein PEEP-Niveau bestimmt (also der kleinere Druck von P1 und P2). Zur Bestimmung des PEEP-Niveaus, mit dem der Patient im Folgenden beatmet werden soll, können zunächst auch mehrere Beatmungsvorgänge mit jeweils unterschiedlichen PEEP-Niveaus durchgeführt werden.

Weiter wird ein für den vorliegenden Patienten erwartetes Atemzugvolumen $V_T$ eingestellt. Dieses Atemzugvolumen $V_T$ kann während der Beatmung z. B. anhand der Überwachung des $CO_2$-Niveaus weiter angepasst werden. Alternativ oder zusätzlich kann das $CO_2$-Niveau auch mittels der Frequenz der Beatmungsvorgänge beeinflusst werden.

Insbesondere wird der Druckanstieg und/oder der Druckabfall während der Beatmung gesteuert und kontrolliert, so dass der auf den mindestens einen Atemweg einwirkende "shear-stress" minimiert wird.

In jedem Fall aber wird durch die Beatmungsvorrichtung und/oder das Verfahren sichergestellt, dass ein Betrag der Compliance während eines Beatmungsvorgangs möglichst groß ist, oder insbesondere in anderen Worten, dass

(1) die Beatmung in einem Druckintervall erfolgt, in dem das zugeführte Volumen des Fluids maximal ist, bzw.

(2) die Zu- oder Abfuhr eines vorbestimmten Volumens V bzw. eines Atemzugvolumens $V_T$ des Fluids innerhalb eines Druckintervalls erfolgt, dass möglichst klein ist.

Es wird ein weiteres (zweites) Verfahren zum Beatmen eines Patienten und/oder zum Betreiben einer Beatmungsvorrichtung, insbesondere der erfindungsgemäßen Beatmungsvorrichtung, vorgeschlagen. Die Beatmungsvorrichtung ist zur Beatmung eines Patienten vorgesehen.

Auch das zweite Verfahren ist auf die Beatmung eines Patienten gerichtet, wobei ein möglichst geringer Energieeintrag in die Atemwege des Patienten erreicht werden soll. Gemäß dem zweiten Verfahren erfolgt während der Beatmung des Patienten eine kontinuierliche (also zu jedem Zeitpunkt stattfindende) aktive Steuerung des während der Inspiration der Lunge des Patienten zugeführten oder während der Exspiration aus der Lunge des Patienten abgeführten Fluids durch die Beatmungsvorrichtung. Die aktive Steuerung umfasst eine kontinuierliche Druckänderung des zugeführten und abgeführten Fluids durch die Beatmungsvorrichtung. Der kontinuierlich geänderte Druck ist insbesondere der Druck innerhalb der Atemwege und damit innerhalb der Lunge. Dieser Druck kann über eine Messung am Ende einer, in den Atemweg hinreichenden Beatmungsvorrichtung, z. B. einem Katheter, über einen Sensor bestimmt werden.

Die kontinuierliche Druckänderung führt insbesondere zu einer kontinuierlichen Steuerung der Fluidzufuhrrate und Fluidabfuhrrate [Milliliter/Sekunde] durch die Beatmungsvorrichtung hin zu der Lunge bzw. aus der Lunge während der Beatmungsvorgänge. Insbesondere wird so das in der Lunge vorhandene Fluidvolumen kontinuierlich geändert. Bevorzugt werden während der Änderung des in der Lunge befindlichen Fluidvolumens die Fluidzufuhrrate und/oder die Fluidabfuhrrate durch die Beatmungsvorrichtung zu der Lunge hin bzw. aus der Lunge nicht verändert und bleibt entsprechend im Wesentlichen konstant. Dabei muss die Fluidzufuhrrate nicht zwingend der Fluidabfuhrrate entsprechen, sie kann aber auch betragsgleich sein. Weiter kann die Fluidzufuhrrate von einem Inspirationsvorgang zum folgenden Inspirationsvorgang variiert werden. Gleiches gilt - insbesondere davon unabhängig - für die Fluidabfuhrrate während aufeinanderfolgender Exspirationsvorgänge.

Insbesondere werden Zustände, in denen innerhalb eines Zeitintervalls keine Änderung des Drucks und insbesondere keine Änderung des in der Lunge vorhandenen Fluidvolumens erfolgen, vermieden. Bevorzugt sind solche Zeitintervalle, in denen keine Änderung des Drucks und/oder insbesondere keine Änderung des in der Lunge vorhandenen Fluidvolumens erfolgen, höchstens 0,5 s [Sekunden], insbesondere höchstens 0,2 s, bevorzugt höchstens 0,1 s lang und betreffen insbesondere (ausschließlich) den Zeitpunkt der Fluidstromumkehr (also Übergang von Fluidzufuhr zu Fluidabfuhr und umgekehrt).

Der Druck wird insbesondere in dem Patienten selbst gemessen, besonders vorteilhaft im Bereich der Ausströmung aus der Beatmungsvorrichtung, also aus einem das Fluid transportierenden Lumen (Tubus/Katheter), in den Atemweg des Patienten. Alternativ und/oder zusätzlich wird der Druck in der Beatmungsvorrichtung gemessen.

Der Druck in der Beatmungsvorrichtung entspricht insbesondere nicht dem Druck in dem Atemweg des Patienten. Insbesondere kann eine kontinuierliche Änderung des Drucks in den Atemwegen auch durch einen zumindest zeitweisen konstanten Druck in der Beatmungsvorrichtung eingestellt werden.

Eine Änderung des Drucks in den Atemwegen kann insbesondere auch dann noch gemessen werden, wenn eine Fluidzufuhrrate oder Fluidabfuhrrate Null beträgt. Diese Änderung resultiert insbesondere aus den Eigenschaften der Atemwege selbst. Im Rahmen des (zweiten) Verfahrens wird jedoch auf den Zusammenhang zwischen Fluidzufuhrrate und Fluidabfuhrrate (ungleich Null) und Druckänderung abgestellt. Eine Fluidzufuhrrate und Fluidabfuhrrate von Null soll möglichst vermieden werden (höchstens für Zeitintervalle von bis zu 0,5 s [Sekunden], insbesondere höchstens 0,2 s oder 0,1 s, und auch dann nur zum Zeitpunkt der Fluidstromumkehr; ggf. sind längere Zeitintervalle von bis zu 2,0 s möglich, um z. B. eine Druckmessung durchzuführen, wobei ein solches verlängertes Zeitintervall nur in Abständen von mindestens 30 s, insbesondere mindestens 2 Minuten, bevorzugt mindestens 5 Minuten vorgesehen wird). Die Fluidzufuhrrate und Fluidabfuhrrate wird dazu insbesondere (ausschließlich) durch die Beatmungsvorrichtung vorgegeben, wobei der Druck in den Atemwegen überwacht wird.

Insbesondere wird so ein sinusförmiges oder sägezahnartiges Beatmungsmuster (Druck [Millibar] über der Zeit [Sekunde]) eingestellt, wobei eine Steigung der Kurve (Druck über der Zeit) ständig ungleich Null ist und insbesondere nur zu dem Zeitpunkt der Fluidstromumkehr für ein Zeitintervall von höchstens 0,5 s [Sekunden], insbesondere höchstens 0,2 s, bevorzugt höchstens 0,1 s, besonders bevorzugt nie, eine Steigung von Null aufweist.

Im Rahmen des zweiten Verfahrens wird dem Patienten bevorzugt zu allen Zeitpunkten der Beatmung durch die Beatmungsvorrichtung ein Beatmungsmuster vorgegeben, d. h. die Fluidzufuhrrate (inspiratorischer Fluss) und Fluidabfuhrrate (exspiratorischer Fluss) wird (allein) durch die Beatmungsvorrichtung (und nicht durch den Patienten) gesteuert und bestimmt.

Insbesondere erfolgt die Fluidzufuhr und ggf. zusätzlich die Fluidabfuhr ausschließlich über die Beatmungsvorrichtung bzw. über mindestens ein, in die Atemwege des Patienten eingeführtes Lumen.

Die kontinuierliche Druckänderung stellt sicher, dass die Fluidzufuhr und Fluidabfuhr nicht zu schnell oder zu langsam erfolgt, und kann so eine Schädigung der Atemwege und insbesondere des Lungengewebes verhindern oder zumindest minimieren.

Weiterhin kann die Fluidzufuhr und Fluidabfuhr, z. B. unter Berücksichtigung einer Compliance der Atemwege (siehe Ausführungen zum ersten Verfahren und zur Beatmungsvorrichtung, die gleichermaßen auf das zweite Verfahren übertragbar sind), in vorteilhaften Druckintervallen (also zwischen einem ersten höheren Druck und einem zweitem niedrigeren Druck) und in einer vorbestimmbaren Beatmungsfrequenz erfolgen.

Insbesondere wird bei dem zweiten Verfahren eine Beatmungspause (also kein Fluidstrom zu oder aus dem Atemweg) von mehr als 0,5 s [Sekunden], insbesondere von mehr als 0,2 s, bevorzugt von mehr als 0,1 s, besonders bevorzugt nie, vermieden. Bei den bekannten Beatmungsverfahren werden solche Beatmungspausen vorgesehen, um einen vorgegebenen Beatmungsrhythmus (Frequenz und/oder Verhältnis von Inspiration und Exspiration) einzuhalten oder um das zuzuführende Fluidvolumen (bei einem vorgegebenen Druck) zu begrenzen. Zudem ergeben sich Beatmungspausen bei den bekannten Beatmungsverfahren auch (zufällig) durch die Eigenschaften der Lunge (z. B. Compliance) bzw. werden durch diese beeinflusst. Beatmungspausen führen jedoch dazu, dass eine Fluidzufuhr bzw. Fluidabfuhr zu anderen Zeitpunkten verstärkt bzw. schneller durchgeführt werden muss (mit höherem Fluidstrom und höherem Energieeintrag in die Atemwege bzw. die Lunge des Patienten).

Dieses Problem wird durch das zweite Verfahren dadurch gelöst, dass Beatmungspausen (weitgehend) vermieden werden und die Fluidzufuhr bzw. Fluidabfuhr zu anderen Zeitpunkten dann mit vermindertem Fluidstrom und somit geringerem Energieeintrag in die Atemwege bzw. die Lunge des Patienten durchgeführt werden kann.

Weiter wird auch eine (zweite) Beatmungsvorrichtung beansprucht, insbesondere die erfindungsgemäße (erste) Beatmungsvorrichtung. Die Beatmungsvorrichtung dient zur Beatmung eines Patienten, und umfasst zumindest eine Fluidzuführeinheit und zusätzlich eine Fluidabführeinheit, die zum Zuführen eines Fluids in zumindest einen Atemweg, also in einen Lungenteil oder in die Lunge, eines Patienten und zum Abführen des Fluids aus diesem Atemweg geeignet ist. Weiter umfasst die Beatmungsvorrichtung eine Steuereinrichtung, die, während einer Beatmung des zumindest einen Atemwegs des Patienten, also dem Zuführen eines Fluids in den zumindest einen Atemweg und dem Abführen des Fluids aus dem zumindest einen Atemweg, zur Regelung der Beatmung geeignet ist, also insbesondere zur Steuerung eines Verlaufs zumindest einer der folgenden Größen: Druck in dem Atemweg des Patienten (z. B. durch Messung in der Beatmungsvorrichtung und ggf. Abschätzen des Drucks im Atemweg; oder durch Messung in dem Atemweg des Patienten), Zufuhrrate des Fluids, Abfuhrrate des Fluids, Volumen in dem Atemweg, etc.

Die Steuereinrichtung regelt zumindest einen Beatmungsvorgang so, dass ein Druck in dem zumindest einen Atemweg zumindest während einer gesamten Exspiration, durch kontinuierliche Steuerung einer Fluidabfuhrrate während des Beatmungsvorgangs, kontinuierlich geändert wird.

Insbesondere regelt die Steuereinrichtung zumindest einen Beatmungsvorgang so, dass zumindest während einer gesamten Inspiration eine im Wesentlichen konstante Fluidzufuhrrate [Milliliter/Sekunde] oder zumindest während einer gesamten Exspiration eine im Wesentlichen konstante Fluidabfuhrrate [Milliliter/Sekunde] vorliegt.

Bevorzugt regelt die Steuereinrichtung zumindest einen Beatmungsvorgang, der zumindest eine Inspiration und eine Exspiration umfasst, so, dass ein Druck in dem zumindest einen Atemweg während des Beatmungsvorgangs kontinuierlich geändert wird.

Insbesondere umfasst eine kontinuierliche Änderung des Druckes, dass der Druck höchstens 0,5 s [Sekunden], insbesondere höchstens 0,2 s, bevorzugt 0,1 s, besonders bevorzugt nie, konstant bleibt. Insbesondere ist der Druck nur dann konstant, wenn zwischen einer Fluidzufuhrrate und einer Fluidabfuhrrate umgeschaltet wird.

Die Ausführungen zu der ersten Beatmungsvorrichtung, der zweiten Beatmungsvorrichtung, der Steuereinrichtung, dem ersten Verfahren und dem zweiten Verfahren sind auf die jeweils anderen Gegenstände der vorliegenden Erfindung jeweils übertragbar.

Es wird ausdrücklich darauf hingewiesen, dass die Steuereinrichtung auch unabhängig von der Beatmungsvorrichtung beansprucht werden kann. Die Steuereinrichtung dient insbesondere der Regelung der Beatmungsvorgänge. Hierdurch wird festgelegt, anhand welcher Größen der Beatmungsvorgang gesteuert wird und welche Parameter (maximaler/minimaler Druck, maximale/minimale Fluidzufuhrrate und Fluidabfuhrrate, etc.) dabei überwacht werden.

Die Erfindung sowie das technische Umfeld werden nachfolgend anhand der Figuren näher erläutert. Es ist darauf hinzuweisen, dass die Figuren eine besonders bevorzugte Ausführungsvariante der Erfindung zeigen, diese jedoch nicht darauf beschränkt ist. Dabei sind gleiche Bauteile in den Figuren mit denselben Bezugszeichen versehen. Es zeigen schematisch:

Fig. 1:    eine Beatmungsvorrichtung und einen Patienten;

Fig. 2:    einen Verlauf einer Compliance-Kurve;

Fig. 3:    die Darstellung von Beatmungsvorgängen in einem Druck-/Zeit-Diagramm;

Fig. 4:    die Darstellung von Beatmungsvorgängen in einem Volumen-/Zeit-Diagramm;

Fig. 5:    die Darstellung von Beatmungsvorgängen in einem Fluidzufuhrrate und Fluidabfuhrrate/Zeit-Diagramm;

Fig. 6:    die Darstellung von Beatmungsvorgängen in einem weiteren Druck-/Zeit-Diagramm; und

Fig. 7:    die Darstellung von Beatmungsvorgängen in einem weiteren Volumen-/Zeit-Diagramm.

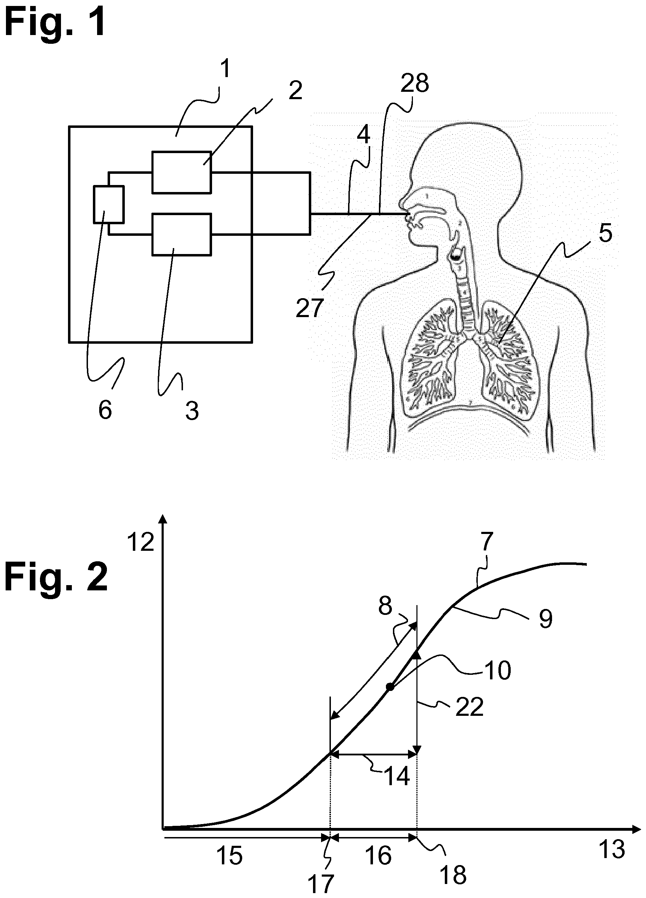

Fig. 1 zeigt eine Beatmungsvorrichtung 1 und einen Patienten mit zumindest einem Atemweg 5, bzw. einer Lunge. Die Beatmungsvorrichtung 1 umfasst eine Fluidzuführeinheit 2 und eine Fluidabführeinheit 3, die zum Zuführen eines Fluids 4 in einen Atemweg 5, also in einen Lungenteil oder in die Lunge, eines Patienten bzw. zum Abführen des Fluids 4 aus diesem Atemweg 5 geeignet sind. Weiter umfasst die Beatmungsvorrichtung 1 eine Steuereinrichtung 6, die, während einer Beatmung des zumindest einen Atemwegs 5 des Patienten, also dem Zuführen eines Fluids 4 in den zumindest einen Atemweg 5 und/oder dem Abführen des Fluids 4 aus dem zumindest einen Atemweg 5 durch Betreiben der Beatmungsvorrichtung 1, zur Ermittlung oder zusätzlich zum Abschätzen eines Verlaufs 7 zumindest eines Teilbereichs 8 einer Compliance-Kurve 9 des mindestens einen Atemwegs 5 durch das Zuführen und/oder Abführen des Fluids 4 aus dem zumindest einen Atemweg 5 und durch Ermittlung zumindest eines Wertes 10 der Compliance 11 geeignet ist. Hier ist die Beatmungsvorrichtung 1 über einen Katheter 27 mit einem von dem Fluid 4 durchströmbaren Querschnitt 28 des Lumens mit dem Atemweg 5 des Patienten verbunden. Die Beatmung erfolgt also z. B. über ein einziges Lumen, insbesondere unter Verwendung einer Gaststromumkehr-Vorrichtung.

Fig. 2 zeigt einen Verlauf 7 einer Compliance-Kurve 9 in einem Druck-Volumen-Diagramm. An der horizontalen Achse ist der Druck 13 aufgetragen, an der vertikalen Achse das Volumen 12. Der Verlauf 7 der Compliance-Kurve 9 ist für jeden Patienten individuell zu ermitteln. Weiter kann sich der Verlauf 7 auch während einer Beatmung verändern.

Im Rahmen des Verfahrens bzw. durch die Beatmungsvorrichtung 1 wird zunächst mindestens ein Wert 10 der Compliance 11 ermittelt, wobei für die Compliance 11 gilt: C = Volumen V 12 / delta p 14 in Milliliter/Millibar. In dem hier gezeigten Teilbereich 8 der Compliance-Kurve 9 ist der Betrag der Compliance 11 maximal. Durch Ermittlung bzw. Abschätzen des Verlaufs 7 der Compliance-Kurve 9 kann nun die Lage 15 eines Druckintervalls 16 mit den Drücken 17, 18 bestimmt werden, in dem ein Atemzugvolumen $V_T$ 22 des Fluids 4 dem mindestens einen Atemweg 5 zugeführt werden kann. Diese Drücke 17, 18 werden an Beatmungsvorrichtung 1 eingestellt, so dass zumindest ein Beatmungsvorgang 19, also eine Inspiration 20 und/oder eine Exspiration 21, jeweils mit einem Atemzugvolumen $V_T$ 22, zwischen diesen Drücken P1 17 und P2 18 erfolgt.

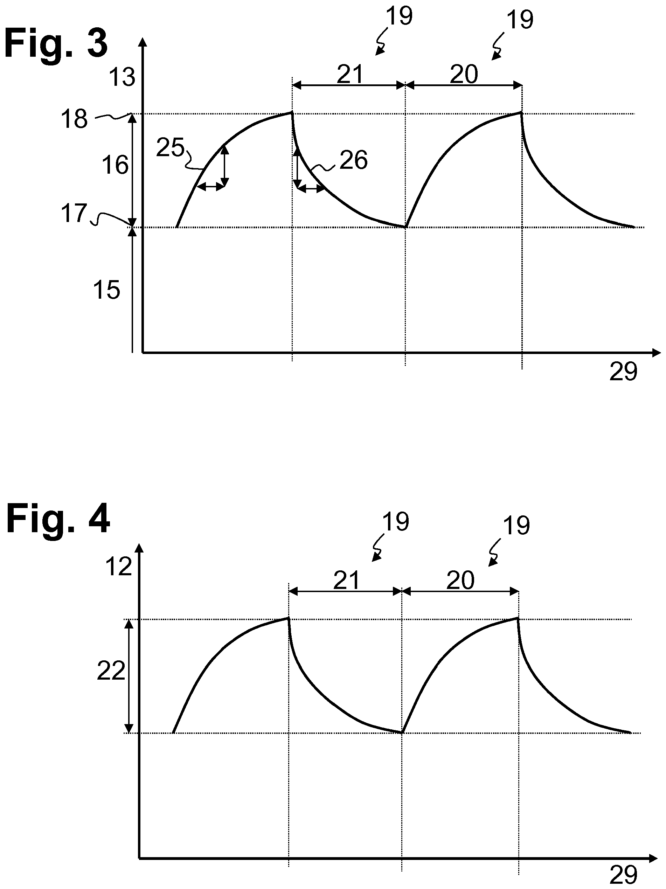

Fig. 3 zeigt die Darstellung von Beatmungsvorgängen 19 in einem Druck-/ZeitDiagramm. Auf der horizontalen Achse ist die Zeit 29 aufgetragen, auf der vertikalen Achse der Druck 13. Die Beatmung erfolgt in einem Druckintervall 16 zwischen den Drücken P1 17 und P2 18. Der Druckanstieg 25, also delta p / delta t, während der Inspiration 20 wird überwacht und kontrolliert. Weiter wird der Druckabfall 26, also delta p / delta t, während der Exspiration 21 überwacht und kontrolliert.

In Fig. 4 sind Beatmungsvorgänge 19 in einem Volumen-/Zeit-Diagramm dargestellt. Auf der horizontalen Achse ist die Zeit 29 aufgetragen, auf der vertikalen Achse das Volumen 12. Das in dem Druckintervall 16 dem Atemweg 5 zugeführte Volumen wird als Atemzugvolumen $V_T$ 22 (Tidalvolumen) bezeichnet. Der Verlauf der Kurve in dem Volumen-/Zeit-Diagramm folgt dem Verlauf des Druckes (siehe Fig. 3).

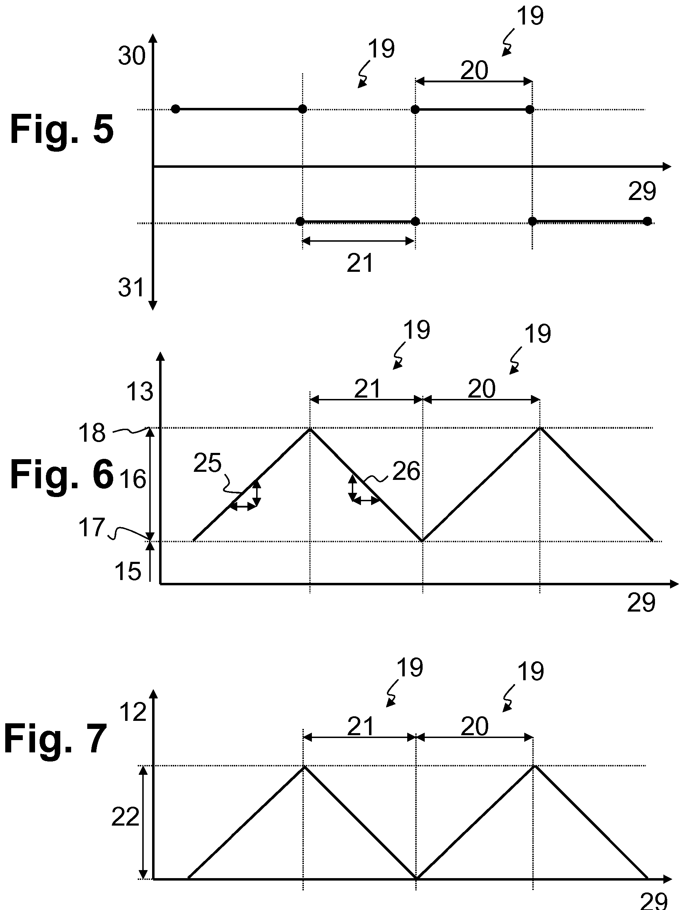

In Fig. 5 sind Beatmungsvorgänge 19 in einem Fluidzufuhrrate und Fluidabfuhrrate/Zeit-Diagramm dargestellt. Auf der horizontalen Achse ist die Zeit 29 aufgetragen, auf der vertikalen Achse die Fluidzufuhrrate 30 (oben, also positiver Wert) bzw. Fluidabfuhrrate 31 (unten, also negativer Wert). Die Fluidzufuhrrate 30 und Fluidabfuhrrate 31 ist jeweils konstant und nie Null. Die Fluidzufuhrrate 30 und Fluidabfuhrrate 31 kann an der Beatmungsvorrichtung 1 durch den Anwender manuell eingestellt oder durch die Steuerlogik der Beatmungsvorrichtung 1 bzw. der Steuereinrichtung 6 automatisch geregelt werden, wobei der Druck 13 überwacht wird. Alternativ kann der Druck 13 (in dem Atemweg 5), wie in Fig. 3 dargestellt, z. B. durch den Anwender eingestellt und z. B. durch die Steuerlogik überwacht werden, so dass sich bei der Beatmungsfrequenz F 23 und/oder vorgegebener Dauer bzw. Zeitverhältnis von Inspiration 20 und Exspiration 21 aufgrund des eingestellten Drucks 13 die gewünschte Fluidzufuhrrate 30 und Fluidabfuhrrate 31 ergibt.

Insbesondere kann die Fluidzufuhrrate 30 und Fluidabfuhrrate 31 über, aus der WO 2008/113752 A1 und WO 2015/004229 A1 bereits bekannte Gasstromumkehrelemente als Beatmungsvorrichtungen 1 eingestellt werden, die maschinell oder manuell betrieben werden können.

Fig. 6 zeigt die Darstellung von Beatmungsvorgängen 19 in einem weiteren Druck-/Zeit-Diagramm. Auf der horizontalen Achse ist die Zeit 29 aufgetragen, auf der vertikalen Achse der Druck 13. Die Beatmung erfolgt in einem Druckintervall 16 mit der Lage 15 zwischen den Drücken P1 17 und P2 18. Der Druckanstieg 25, also delta p / delta t, während der Inspiration 20 wird überwacht und kontrolliert. Weiter wird der Druckabfall 26, also delta p / delta t, während der Exspiration 21 überwacht und kontrolliert. Erkennbar ist hier die Steigung des Druckverlaufs sowohl während der Inspiration 20 als auch während der Exspiration 21 konstant.

Fig. 7 zeigt die Darstellung von Beatmungsvorgänge 19 in einem weiteren Volumen-/Zeit-Diagramm. Auf der horizontalen Achse ist die Zeit 29 aufgetragen, auf der vertikalen Achse das Volumen 12. Das in dem Druckintervall 16 dem Atemweg 5 zugeführte Volumen wird als Atemzugvolumen $V_T$ 22 (Tidalvolumen) bezeichnet. Der Verlauf der Kurve in dem Volumen-/Zeit-Diagramm folgt dem Verlauf des Druckes (siehe Fig. 6). Erkennbar ist auch hier die Steigung des Volumenverlaufs sowohl während der Inspiration 20 als auch während der Exspiration 21 konstant.

Aus den Beatmungsvorgängen 19 in den Fig. 3 bis 7 ist erkennbar, dass keine Beatmungspausen eingelegt werden. Es wird jeweils ohne Pause zwischen Inspiration und Exspiration gewechselt.

**Bezugszeichenliste**

| | |
|---|---|
| 1 | Beatmungsvorrichtung |
| 2 | Fluidzuführeinheit |
| 3 | Fluidabführeinheit |
| 4 | Fluid |
| 5 | Atemweg |
| 6 | Steuereinrichtung |
| 7 | Verlauf |
| 8 | Teilbereich |
| 9 | Compliance-Kurve |
| 10 | Wert |
| 11 | Compliance C |
| 12 | Volumen V |
| 13 | Druck |
| 14 | Druckdifferenz delta p |
| 15 | Lage |
| 16 | Druckintervall |
| 17 | Druck P1 |
| 18 | Druck P2 |
| 19 | Beatmungsvorgang |
| 20 | Inspiration |
| 21 | Exspiration |
| 22 | Atemzugvolumen $V_T$ |
| 23 | Beatmungsfrequenz F |
| 24 | Drucksensor |
| 25 | Druckanstieg |
| 26 | Druckabfall |
| 27 | Katheter |
| 28 | Querschnitt |
| 29 | Zeit |
| 30 | Fluidzufuhrrate |
| 31 | Fluidabfuhrrate |

**Patentansprüche**

1. Beatmungsvorrichtung (1) zur Beatmung eines Patienten, zumindest umfassend eine Fluidzuführeinheit (2) und zusätzlich eine Fluidabführeinheit (3), die zum Zuführen eines Fluids (4) in zumindest einen Atemweg (5), also in einen Lungenteil oder in die Lunge, eines Patienten und zum Abführen des Fluids (4) aus diesem Atemweg (5) geeignet ist; sowie eine Steuereinrichtung (6), die, während einer Beatmung des zumindest einen Atemwegs (5), also dem Zuführen des Fluids (4) in den zumindest einen Atemweg (5) und/oder dem Abführen des Fluids (4) aus dem zumindest einen Atemweg (5) durch Betreiben der Beatmungsvorrichtung (1), zur Ermittlung oder zusätzlich zum Abschätzen eines Verlaufs (7) zumindest eines Teilbereichs (8) einer Compliance-Kurve (9) des zumindest einen Atemwegs (5) durch das Zuführen und/oder Abführen des Fluids (4) aus dem zumindest einen Atemweg (5) und durch Ermittlung zumindest eines Wertes (10) der Compliance (11) geeignet ist; wobei für die Compliance C (11) gilt:

$$C = V \,/\, delta\ p\ [Milliliter/Millibar];$$

wobei die Compliance (11) angibt, wie viel Fluid (4), also ein Volumen V (12) [Milliliter], in den zumindest einen Atemweg (5) eingeführt oder aus dem Atemweg (5) entfernt wird, so dass sich ein Druck (13) in dem Atemweg (5) um eine Druckdifferenz delta p (14) [Millibar] verändert; wobei die Steuereinrichtung (6) unter Berücksichtigung des ermittelten oder zusätzlich abgeschätzten Verlaufs (7) des zumindest einen Teilbereichs (8) der Compliance-Kurve (9) eine Lage (15) eines Druckintervalls (16) mit den Drücken P1 (17) und P2 (18) bestimmt und an der Beatmungsvorrichtung (1) einstellt, so dass zumindest ein Beatmungsvorgang (19), also eine Inspiration (20) und eine Exspiration (21), zwischen diesen Drücken P1 (17) und P2 (18) erfolgt und ein Betrag der Compliance (11) dieses Beatmungsvorgangs (19) möglichst groß ist; **dadurch gekennzeichnet, dass** zu allen Zeitpunkten der Beatmung eine Fluidzuführrate und eine Fluidabfuhrrate allein durch die Beatmungsvorrichtung (1) steuerbar und bestimmbar ist; wobei die Exspiration nicht passiv erfolgt, also angetrieben durch die in den elastischen Gewebeelementen von Lunge und Thorax gespeicherte Energie, sondern während der Beatmung eine kontinuierliche aktive Steuerung des zumindest während der Exspiration aus der Lunge des Patienten abgeführten Fluids durch die Beatmungsvorrichtung (1) erfolgt, wobei die aktive Steuerung eine kontinuierliche Druckänderung des abgeführten Fluids umfasst; wobei die kontinuierliche Druckänderung zu einer kontinuierlichen Steuerung der Fluidabfuhrrate führt.

2. Beatmungsvorrichtung (1) nach Patentanspruch 1, wobei die Steuereinrichtung (6) zumindest während einer Inspiration (20) oder einer Exspiration (21) eines Beatmungsvorgangs (19) eine Mehrzahl von Werten (10) für die Compliance (11) ermittelt und daraus für zumindest einen folgenden Beatmungsvorgang (19) die Lage (15) des Druckintervalls (16) mit den Drücken P1 (17) und P2 (18) bestimmt, für das ein Betrag der Compliance (11) möglichst groß ist.

3. Beatmungsvorrichtung (1) nach einem der vorhergehenden Patentansprüche, wobei die Steuereinrichtung (6) Werte (10) für die Compliance (11) kontinuierlich ermittelt, so dass wahlweise für jeden folgenden Beatmungsvorgang (19) oder für mehrere aufeinanderfolgende Beatmungsvorgänge (19) die Lage (15) des Druckintervalls (16) mit den Drücken P1 (17) und P2 (18) neu bestimmbar ist.

**4.** Beatmungsvorrichtung (1) nach einem der vorhergehenden Patentansprüche, wobei die Steuereinrichtung (6), in Abhängigkeit von der ermittelten Lage (15) des Druckintervalls (16), des Druckintervalls (16) selbst und der dabei bestimmten Compliance (11), zumindest für den folgenden Beatmungsvorgang (19) zumindest einen der folgenden Parameter bestimmt:

> - ein Atemzugvolumen (Tidalvolumen) $V_T$ (22) [Milliliter],
> - einen Druck P1 (17) und einen Druck P2 (18) [Millibar],
> - eine Beatmungsfrequenz F (23) [1/Sekunde].

**5.** Beatmungsvorrichtung (1) nach einem der vorhergehenden Patentansprüche, wobei ein Drucksensor (24) innerhalb des zumindest einen Atemwegs (5) anordenbar ist und der Druck (13) über eine Messung innerhalb des zumindest einen Atemwegs (5) ermittelbar ist.

**6.** Beatmungsvorrichtung (1) nach einem der vorhergehenden Patentansprüche, wobei durch die Beatmungsvorrichtung (1) zumindest ein Druckanstieg (25), also delta p / delta t [Millibar/Sekunde] während einer Inspiration (20) kontrollierbar und begrenzbar ist.

**7.** Beatmungsvorrichtung (1) nach einem der vorhergehenden Patentansprüche, wobei durch die Beatmungsvorrichtung (1) zumindest ein Druckabfall (26), also delta p / delta t [Millibar/Sekunde] während einer Exspiration (21) kontrollierbar und begrenzbar ist.

**8.** Beatmungsvorrichtung (1) nach Patentanspruch 6 oder 7, wobei der Betrag des Druckanstiegs (25) oder Druckabfalls (26) auf höchstens 40 mbar/s [Millibar/Sekunde] begrenzbar ist.

**9.** Beatmungsvorrichtung (1) zur Beatmung eines Patienten, zumindest umfassend eine Fluidzuführeinheit (2) und zusätzlich eine Fluidabführeinheit (3), die zum Zuführen eines Fluids (4) in zumindest einen Atemweg (5), also in einen Lungenteil oder in die Lunge, eines Patienten und zum Abführen des Fluids (4) aus diesem Atemweg (5) geeignet ist; sowie eine Steuereinrichtung (6), die während einer Beatmung des zumindest einen Atemwegs (5), also dem Zuführen des Fluids (4) in den zumindest einen Atemweg (5) und dem Abführen des Fluids (4) aus dem zumindest einen Atemweg (5) durch Betreiben der Beatmungsvorrichtung (1), zur Regelung der Beatmung geeignet ist; wobei die Steuereinrichtung (6) zumindest einen Beatmungsvorgang (19) so regelt, dass ein Druck (13) in dem zumindest einen Atemweg (5) zumindest während einer gesamten Exspiration (21), durch kontinuierliche Steuerung einer Fluidabfuhrrate (31) während des Beatmungsvorgangs (19), kontinuierlich geändert wird; **dadurch gekennzeichnet, dass** zu allen Zeitpunkten der Beatmung eine Fluidzuführrate und eine Fluidabfuhrrate allein durch die Beatmungsvorrichtung (1) steuerbar und bestimmbar ist; wobei die Exspiration nicht passiv erfolgt, also angetrieben durch die in den elastischen Gewebeelementen von Lunge und Thorax gespeicherte Energie, sondern während der Beatmung eine kontinuierliche aktive Steuerung des zumindest während der Exspiration aus der Lunge des Patienten abgeführten Fluids durch die Beatmungsvorrichtung (1) erfolgt, wobei die aktive Steuerung eine kontinuierliche Druckänderung des abgeführten Fluids umfasst; wobei die kontinuierliche Druckänderung zu einer kontinuierlichen Steuerung der Fluidabfuhrrate führt.

**10.** Beatmungsvorrichtung (1) nach Patentanspruch 9, wobei die Steuereinrichtung (6) zumindest einen Beatmungsvorgang (19) so regelt, dass zumindest während einer gesamten Inspiration (20) eine im Wesentlichen konstanten Fluidzufuhrrate (30) [Milliliter/Sekunde] oder zumindest während einer gesamten Exspiration (21) eine im Wesentlichen konstante Fluidabfuhrrate (31) [Milliliter/Sekunde] vorliegt.

**11.** Beatmungsvorrichtung (1) nach einem der Patentansprüche 9 oder 10, wobei die Steuereinrichtung (6) zumindest einen Beatmungsvorgang (19), der zumindest eine Inspiration (20) und eine Exspiration (21) umfasst, so regelt, dass ein Druck (13) in dem zumindest einen Atemweg (5) während des Beatmungsvorgangs (19) kontinuierlich geändert wird.

**12.** Beatmungsvorrichtung (1) nach Patentanspruch 11, wobei eine kontinuierliche Änderung des Druckes (13) umfasst, dass der Druck (13) beim Übergang zwischen Fluidzufuhr und Fluidabfuhr höchstens 0,5 s [Sekunden] konstant bleibt.

**Claims**

**1.** Ventilation device (1) for ventilating a patient, at least comprising a fluid supply unit (2) and, additionally, a fluid discharge unit (3), which is suitable for supplying a fluid (4) into at least one airway (5), that is to say into a lung part or into the lung, of a patient and for discharging the fluid (4) from this airway (5); and a control device (6) which, during a ventilation of the at least one airway (5), that is to say during the supply of the fluid (4) into the at least one airway (5) and/or the discharge of the fluid (4) from the at least one airway (5) by operation of the ventilation device (1), is suitable for determining or in addition for estimating a profile (7) of at least one subregion (8) of a compliance curve (9) of the at least one airway (5) by the supply and/or discharge of the fluid (4) from the at least one airway (5) and by determination of at least one value (10) of the compliance (11); wherein the following applies for the compliance C (11):

$$C = V \,/\, \text{delta p [milliliter/millibar]};$$

wherein the compliance (11) indicates how much fluid (4), that is to say a volume V (12) [milliliter], is introduced into the at least one airway (5) or is removed from the airway (5), such that a pressure (13) in the airway (5) changes by a pressure difference delta p (14) [millibar]; wherein the control device (6), taking into account the determined or additionally estimated profile (7) of the at least one subregion (8) of the compliance curve (9), determines a position (15) of a pressure interval (16) with the pressures P1 (17) and P2 (18) and sets the ventilation device (1) such that at least one ventilation process (19), that is to say an inhalation (20) and an exhalation (21), takes place between these pressures P1 (17) and P2 (18) and an absolute value of the compliance (11) of this ventilation process (19) is as high as possible, **characterized in that** at all times during the ventilation a fluid supply rate and a fluid discharge rate are controllable and determinable by the ventilation device alone; wherein the exhalation does not take place passively, i.e. the energy stored in the elastic tissue elements of lung and thorax drives the exhalation; but that during ventilation a continuous active control of at least the during the exhalation from the patient's lung discharged fluid takes place, wherein the active control comprises a continuous pressure change of the discharged fluid; wherein the continuous pressure change leads to continuous control of the fluid discharge rate.

2. Ventilation device (1) as claimed in claim 1, wherein the control device (6), at least during an inhalation (20) or an exhalation (21) of a ventilation process (19), determines a plurality of values (10) for the compliance (11) and from these determines, for at least one subsequent ventilation process (19), the position (15) of the pressure interval (16) with the pressures P1 (17) and P2 (18), for which an absolute value of the compliance (11) is as high as possible.

3. Ventilation device (1) as claimed in one of the preceding claims, wherein the control device (6) continuously determines values (10) for the compliance (11), such that the position (15) of the pressure interval (16) with the pressures P1 (17) and P2 (18) can be newly determined optionally for each subsequent ventilation process (19) or for a plurality of successive ventilation processes (19).

4. Ventilation device (1) as claimed in one of the preceding claims, wherein the control device (6), as a function of the determined position (15) of the pressure interval (16), of the pressure interval (16) itself and of the determined compliance (11), determines at least for the subsequent ventilation process (19) at least one of the following parameters:

   - a tidal volume $V_T$ (22) [milliliter],
   - a pressure P1 (17) and a pressure P2 (18) [millibar],
   - a ventilation frequency F (23) [1/second].

5. Ventilation device (1) as claimed in one of the preceding claims, wherein a pressure sensor (24) can be arranged inside the at least one airway (5), and the pressure (13) can be determined by a measurement inside the at least one airway (5).

6. Ventilation device (1) as claimed in one of the preceding claims, wherein at least one pressure rise (25), that is to say delta p/delta t [millibar/ second], during an inhalation (20) can be controlled and limited by the ventilation device (1).

7. Ventilation device (1) as claimed in one of the preceding claims, wherein at least one pressure drop (26), that is to say delta p/delta t [millibar/ second], during an exhalation (21) can be controlled and limited by the ventilation device (1).

8. Ventilation device (1) as claimed in claim 6 or 7, wherein the absolute value of the pressure rise (25) or pressure drop (26) can be limited to at most 40 mbar/s [millibar/second].

9. Ventilation device (1) for ventilating a patient, at least comprising a fluid supply unit (2) and, additionally, a fluid discharge unit (3), which is suitable for supplying a fluid (4) into at least one airway (5), that is to say into a lung part or into the lung, of a patient and for discharging the fluid (4) from this airway (5); and a control device (6) which, during a ventilation of the at least one airway (5), that is to say during the supply of the fluid (4) into the at least one airway (5) and the discharge of the fluid (4) from the at least one airway (5) by operation of the ventilation device (1), is suitable for regulating the ventilation; wherein the control device (6) regulates at least one ventilation process (19) such that a pressure (13) in the at least one airway (5) is continuously changed at least during an entire exhalation (21), by continuous control of a fluid discharge rate (31) during the ventilation process (19); **characterized in that** at all times during the ventilation a fluid supply rate and a fluid discharge rate are controllable and determinable by the ventilation device alone; wherein the exhalation does not take place passively, i.e. the energy stored in the elastic tissue elements of lung and thorax drives the exhalation; but that during ventilation a continuous active control of at least the during the exhalation from the patient's lung discharged fluid takes place, wherein the active control comprises a continuous pressure change of the discharged fluid; wherein the continuous pressure change leads to continuous control of the fluid discharge rate.

10. Ventilation device (1) as claimed in claim 9, wherein the control device (6) regulates at least one ventilation process (19) such that there is a substantially constant fluid supply rate (30) [milliliter/second] at least during an entire inhalation (20) or there is a substantially constant fluid discharge rate (31) [milliliter/second] at least during an entire exhalation (21).

11. Ventilation device (1) as claimed in either of claims 9 and 10, wherein the control device (6) regulates at least one ventilation process (19), which comprises at least one inhalation (20) and one exhalation (21), such that a pressure (13) in the at least one airway (5) is changed continuously during the ventilation process (19).

12. Ventilation device (1) as claimed in claim 11, wherein a continuous change of the pressure (13) entails that the pressure (13) at the time of the transition from fluid supply to fluid discharge remains constant for at most 0.5 s [seconds] .

**Revendications**

1. Dispositif de ventilation (1) destiné à la ventilation d'un patient, ledit dispositif comprenant au moins une unité d'alimentation en fluide (2) et en plus une unité d'évacuation de fluide (3) et étant approprié pour amener un fluide (4) dans au moins une voie respiratoire (5), c'est-à-dire dans une partie du poumon ou dans les poumons, d'un patient et pour évacuer le fluide (4) de cette voie respiratoire (5) ; et un moyen de commande (6) qui est approprié, pendant la ventilation de l'au moins une voie respiratoire (5), c'est-à-dire l'amenée du fluide (4) dans l'au moins une voie respiratoire (5) et/ou l'évacuation du fluide (4) de l'au moins une voie respiratoire (5) par le fonctionnement du dispositif de ventilation (1), pour déterminer ou en plus pour estimer une variation (7) d'au moins une partie (8) d'une courbe de compliance (9) de l'au moins une voie respiratoire (5) par l'amenée et/ou l'évacuation du fluide (4) dans respectivement l'au moins une voie respiratoire (5) et par détermination de l'au moins une valeur (10) de la compliance (11) ; pour la compliance C (11), on a :

$$C = V/\text{delta } p \ [\text{millilitre/millibar}] \ ;$$

la compliance (11) indiquant la quantité de fluide (4), c'est-à-dire un volume V (12) [millilitre], introduite dans l'au moins une voie respiratoire (5) ou évacuée de la voie respiratoire (5) de sorte qu'une pression (13) varie dans la voie respiratoire (5) d'une différence de pression delta p (14) [millibars] ;
le moyen de commande (6) déterminant, en prenant en compte la variation (7) déterminée ou en plus estimée de l'au moins une partie (8) de la courbe de compliance (9), une position (15) d'un intervalle de pression (16) avec les pressions P1 (17) et P2 (18) et réglant celle-ci au niveau du dispositif de ventilation (1) de sorte qu'au moins un processus de ventilation (19), c'est-à-dire une inspiration (20) et une expiration (21), soit effectuée entre ces pressions P1 (17) et P2 (18) et la valeur de compliance (11) de ce processus de ventilation (19) étant aussi grande que possible ; **caractérisé en ce qu'**un débit d'alimentation en fluide et un débit d'évacuation de fluide peuvent être commandés et déterminés uniquement par le dispositif de ventilation (1) à tout moment de la ventilation ; l'expiration n'étant pas effectuée de manière passive, c'est-à-dire n'étant pas entraînée par l'énergie stockée dans les éléments tissulaires élastiques des poumons et du thorax, mais une commande active continue du fluide évacué des poumons du patient, au moins pendant l'expiration, étant effectuée par le dispositif de ventilation (1) pendant la ventilation, la commande active comprenant une variation de pression continue du fluide évacué ; la variation de pression continue se traduisant par une commande continue du débit d'évacuation de fluide.

2. Dispositif de ventilation (1) selon la revendication 1, le moyen de commande (6) déterminant une pluralité de valeurs (10) de la compliance (11) au moins pendant une inspiration (20) ou une expiration (21) d'un processus de ventilation (19) et à partir de là, pour l'au moins un processus de ventilation suivant (19), la position (15) de l'intervalle de pression (16), pour lequel un degré de compliance (11) est aussi grand que possible, étant déterminée avec les pressions P1 (17) et P2 (18).

3. Dispositif de ventilation (1) selon l'une des revendications précédentes, le moyen de commande (6) déterminant en continu des valeurs (10) de la compliance (11) de sorte que la position (15) de l'intervalle de pression (16) peut à nouveau déterminée avec les pressions P1 (17) et P2 (18) au choix pour chaque processus de ventilation suivant (19) ou pour plusieurs processus de ventilation successifs (19).

4. Dispositif de ventilation (1) selon l'une des revendications précédentes, le moyen de commande (6) déterminant, en fonction de la position déterminée (15) de l'intervalle de pression (16), de l'intervalle de pression (16) lui-même et de la compliance (11) ainsi déterminée, au moins pour le processus de ventilation suivant (19), l'un au moins des paramètres suivants :

   - un afflux volumique (volume courant) $V_T$ (22) [millilitre],
   - une pression P1 (17) et une pression P2 (18) [millibar],
   - une fréquence de ventilation F (23) [1/seconde].

5. Dispositif de ventilation (1) selon l'une des revendications précédentes, un capteur de pression (24) pouvant être disposé dans l'au moins une voie respiratoire (5) et la pression (13) pouvant être déterminée par une mesure dans l'au moins une voie respiratoire (5).

6. Dispositif de ventilation (1) selon l'une des revendications précédentes, au moins une augmentation de pression (25), c'est-à-dire delta p/delta t [millibars/seconde], pouvant être commandée et limitée par le dispositif de ventilation (1) lors d'une inspiration (20).

7. Dispositif de ventilation (1) selon l'une des revendications précédentes, au moins une baisse de pression (26), c'est-à-dire delta p/delta t [millibars/seconde], pouvant être commandée et limitée par le dispositif de ventilation (1) lors de l'expiration (21).

8. Dispositif de ventilation (1) selon la revendication 6 ou 7, la valeur de l'augmentation de pression (25) ou de la baisse de pression (26) pouvant être limitée à un maximum de 40 mbar/s [millibars/seconde].

9. Dispositif de ventilation (1) destiné à la ventilation d'un patient, ledit dispositif comprenant au moins une unité d'alimentation en fluide (2) et en plus une unité d'évacuation de fluide (3) et étant approprié pour amener un fluide (4) dans au moins une voie respiratoire (5), c'est-à-dire dans une partie du poumon ou dans les poumons, d'un patient et pour évacuer le fluide (4) de cette voie respiratoire (5) ; et un moyen de commande (6) qui est appropriée, pendant la ventilation de l'au moins une voie respiratoire (5), c'est-à-dire l'amenée du fluide (4) dans l'au moins une voie respiratoire (5) et/ou l'évacuation du fluide (4) de l'au moins une voie respiratoire (5) par le fonctionnement du dispositif de ventilation (1), pour réguler la ventilation ; le moyen de commande (6) régulant au moins un processus de ventilation (19) de façon à faire varier en continu une pression (13) dans l'au moins une voie respiratoire (5) au moins pendant toute l'expiration (21) en commandant en continu un débit d'évacuation de fluide (31) pendant le processus de ventilation (19) ; **caractérisé en ce qu'**un débit d'alimentation en fluide et un débit d'évacuation de fluide peuvent être commandés et déterminés uniquement par le dispositif de ventilation (1) à tout moment de la ventilation ; l'expiration n'étant pas effectuée de manière passive, c'est-à-dire n'étant pas entraînée par l'énergie stockée dans les éléments tissulaires élastiques des poumons et du thorax, mais une commande active continue du fluide évacué des poumons du patient, au moins pendant l'expiration, étant effectuée par le dispositif de ventilation (1) pendant la ventilation, la commande active comprenant une variation de pression continue du fluide évacué ; la variation de pression continue se traduisant par une commande continue du débit d'évacuation de fluide.

10. Dispositif de ventilation (1) selon la revendication 9, le moyen de commande (6) régulant au moins un processus de ventilation (19) de façon à avoir un débit d'alimentation en fluide sensiblement constant (30) [millilitres/seconde] au moins pendant toute une inspiration (20) ou un débit d'évacuation de fluide sensiblement constant (31) [millilitres/seconde] pendant toute une expiration (21).

11. Dispositif de ventilation (1) selon l'une des revendications 9 et 10, le moyen de commande (6) régulant au moins un processus de ventilation (19), qui comprend au moins une inspiration (20) et une expiration (21), de façon à faire varier en continu une pression (13) dans l'au moins une voie respiratoire (5) pendant le processus de ventilation (19).

12. Dispositif de ventilation (1) selon la revendication 11, une variation continue de la pression (13) comprenant le fait que la pression (13) reste constante lors de la transition entre l'amenée de fluide et l'évacuation de fluide pendant un maximum de 0,5 s [secondes].

# Fig. 1

# Fig. 2

Fig. 3

Fig. 4

**Fig. 5**

**Fig. 6**

**Fig. 7**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008113752 A1
- WO 2015004229 A1
- EP 0776672 A1
- US 20100108066 A1